# EUROPEAN PATENT SPECIFICATION

(11) **EP 3 998 997 B1**
(45) Date of publication and mention of the grant of the patent: **09.09.2026**
(21) Application number: 20750742.7
(22) Date of filing: 17.07.2020
(51) Int. Cl.: A61F 2/32, A61F 2/38, A61F 2/40

(54) **SPACER DEVICE FOR THE CONTROL OF DISTURBANCES OR INFECTIONS**
ABSTANDSHALTERVORRICHTUNG ZUR BEKÄMPFUNG VON STÖRUNGEN ODER INFEKTIONEN
DISPOSITIF D'ESPACEMENT POUR LE CONTRÔLE DE PERTURBATIONS OU D'INFECTIONS

(30) Priority: 19.07.2019 IT 201900012372
(43) Date of publication of application: 25.05.2022
(73) Proprietor: Tecres S.p.A., 37066 Sommacampagna (VR) (IT)
(72) Inventor: FACCIOLI, Giovanni, 37066 Sommacampagna (Verona) (IT); SOFFIATTI, Renzo, 37066 Sommacampagna (Verona) (IT)
(74) Representative: Feltrinelli, Secondo Andrea
(86) International application number: PCT/IB2020/056752
(87) International publication number: WO 2021/014313

(56) References cited:
- US-A1- 2010 204 802
- US-A1- 2011 319 755
- US-A1- 2016 235 955
- US-A1- 2018 243 556

## Description

### TECHNICAL FIELD OF THE INVENTION

The present invention relates to a spacer device added with antibiotics for the control of ailments or infections, in particular a spacer device for the knee, hip, elbow or shoulder.

### STATE OF THE PRIOR ART

Many joint spacers have been proposed, which are usually made of bone cement.

As it is known, these spacers are used to treat infections occurring after a prosthesis has been grafted, so they are often added with antibiotics.

However, sometimes the infection, even in the presence of spacers, is not treated or is treated very slowly and it is difficult to verify whether this occurs or not, if not when the spacer is removed or if the patient suffers from ailments or pain.

Moreover, in particular with reference to knee and hip spacers, patients sometimes report pain, which can often be attributed to incorrect positioning of the spacer or incorrect preparation of the implant site of the spacer, problems that can be detected only upon patient reporting.

US2016235955A1, which defines the preamble of the independent claims, US2011319755A1, US2018243556A1. US2010204802A1 disclose respective solutions according to the state of the art.

### OBJECTS OF THE INVENTION

An object of the present invention is to provide a new spacer device. Another object of the present invention is to provide a spacer device which allows to detect useful parameters regarding the implant site or the spacer itself as soon as implanted.

Another object of the present invention is to provide a spacer device that makes it possible to detect the permanence or healing of an infection and the possible course of the same.

Another object of the present invention is to provide a spacer device capable of performing a diagnostic activity.

Another object of the present invention is to provide a spacer device that allows to detect an incorrect positioning of the spacer or an incorrect preparation of the implant site thereof.

Another object of the present invention is to provide a spacer device that allows to detect the permanence or healing of an infection, incorrect positioning of the spacer or incorrect preparation of the implant site in a simple, quick and reliable way.

Another object of the present invention is to provide a spacer device as indicated above which does not involve risks for the health of a patient.

Another object of the present invention is to provide a new unit for detecting patient parameters or ailments.

In accordance with an aspect of the invention, a spacer device according to the independent claims is provided.

The dependent claims refer to preferred and advantageous embodiments of the invention.

### BRIEF DESCRIPTION OF THE DRAWINGS

Other features and advantages of the invention will be more evident from the description of an embodiment of a spacer device and a unit, illustrated by way of example in the accompanying drawings in which:
- figure 1 is a slightly top perspective view of a spacer device according to the present invention, in particular a tibial knee spacer device,
- figures 2 to 5 are front, bottom with transparent part, side and top views, respectively, of the spacer of figure 1,
- figure 6 is a section view along the line VI-VI of figure 4,
- figures 7 and 8 are exploded views of the spacer of figure 1,

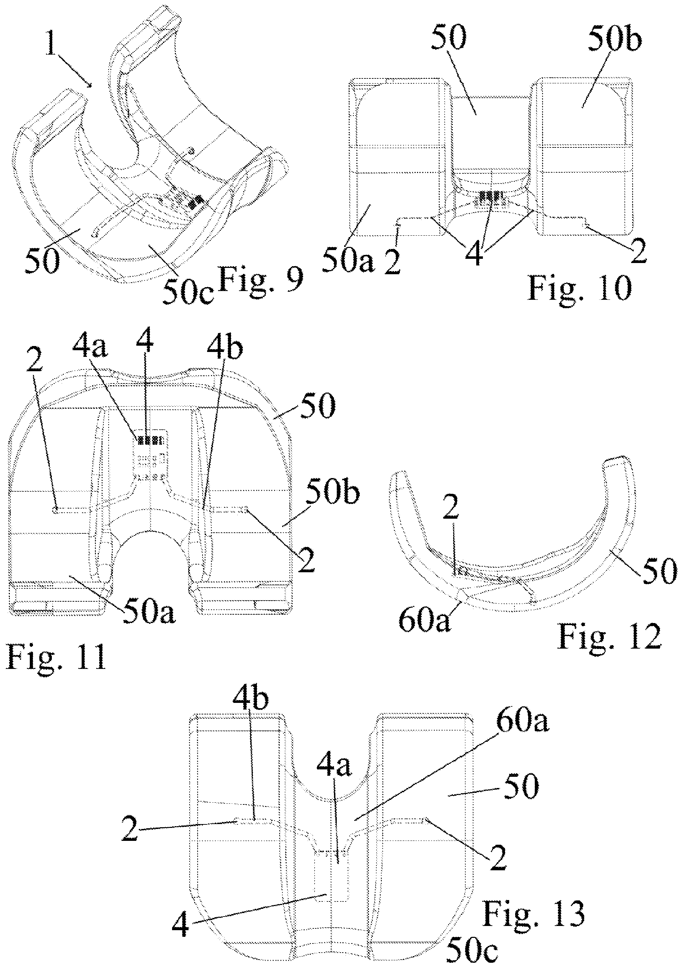

- figures 9 to 13 are perspective slightly from above, rear, top, side and bottom with parts in transparency, respectively, of another spacer device according to the present invention, in particular a knee femoral spacer device,
- figure 14 is a perspective view of another spacer according to the present invention, in particular a femoral hip spacer device,
- figure 15 is a view of a detail on an enlarged scale of figure 10,
- figures 16 to 18 are side, rear and top views, respectively, of the spacer of figure 14,
- figures 19 to 22 are perspective on one side, side, plan, and perspective on the other side with parts in transparency views, respectively, of a further spacer device according to the present invention, in particular an hip acetabular cup spacer device,

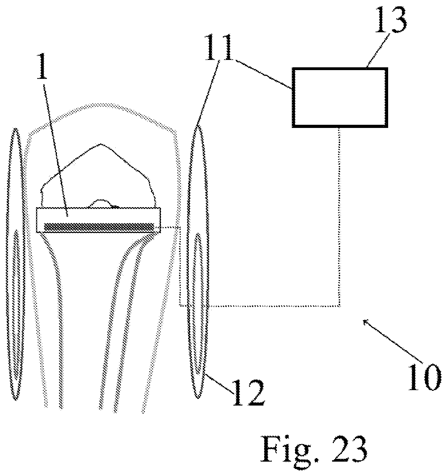

- figure 23 is a schematic view of a detection unit according to the present invention.

In the accompanying drawings, identical parts or components are identified by the same reference numbers.

### EMBODIMENTS OF THE INVENTION

With reference to the attached figures, a spacer device 1 according to the present invention has been illustrated which is intended to be temporarily implanted at a patient's joint area for the replacement of a joint prosthesis, for example infected or extracted from an infected area, for preservation of the dimensions or spaces of the patient's joint area before the implantation of a new prosthesis, in particular after the treatment of the infected area.

A spacer device according to the present invention can for example be a knee, hip, shoulder, ankle or elbow spacer added with antibiotics.

The spacer device 1 comprises at least a sensor 2, 3 as well as electronic means 4 for obtaining and, if desired, transmitting the data detected by the sensor 2, 3, which electronic means 4 are clearly in electrical/electronic communication with the sensor(s) 2, 3.

The sensors or transducers 2, 3 can be of a mechanical, electromechanical, electronic, electrochemical, biochemical or bioelectronic nature. Thus, for example, a mechanical sensor could be used as a temperature detector, if desired including a thermocouple formed by the junction of two metal wires (R, S, B, J, T, E, K, N, C and A types) or of the type electromechanical as an electronic semiconductor resistance thermometer, a transistor or of the electrochemical type, such as liquid crystals.

The electronic means 4 can perform the functions of patient data detection, processing, storage, transmission and reception.

If desired, the electronic means 4 comprise wireless data transmission components.

The expression electronic means 4 means both an optional board 4a or the like, if desired for wireless transmitting, and the electrical connection wires 4b between the board 4a and the sensors 2, 3.

Moreover, detection means of a possible infection in the spacer device 1 or in the implanting area of the same can be provided in the spacer, which detection means comprise at least one temperature sensor 2.

In this regard, an infection always causes an increase in the local temperature.

As it will be understood, the temperature is a very important parameter in particular at night, when there is no friction due to movement and the temperature detected by the sensor is only a function of the possible inflammation given by the infection.

Thanks to this expedient, a forecast of the presence of inflammation is obtained before removing the spacer, so that it is possible to understand whether the inflammation is being treated or not.

Moreover, as a function of this, a spacer device according to the present invention would be able to perform a diagnostic activity, since it can actually help the doctor to diagnose the progress or development of the effectiveness of the spacer in eliminating or reducing an infection.

In essence, the temperature sensor 2 completes and integrates the action of a spacer device 1, since the latter is inserted to maintain the joint spaces and to heal an ongoing infection by releasing locally antibiotics and a temperature sensor as indicated above makes it possible to monitor if the infection is still present.

Furthermore, the status or possible evolution, improvement or worsening of the infection could also be evaluated according to the temperature, since clearly if a temperature decrease is detected over time, it means that the infection is healing, even if the temperature has not fallen yet below a threshold value indicating the absence of infection.

The temperature sensor 2 can be very small in size, for example with a width of about 2-5 mm.

The temperature sensor 2 can comprise a resistance thermometer or a semiconductor or a thermocouple, for example of type K.

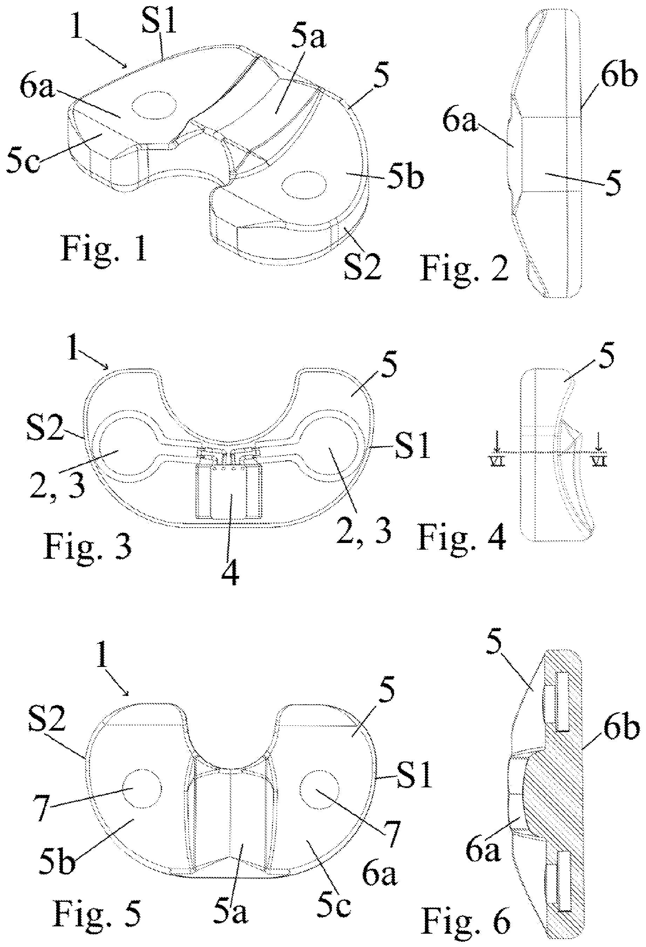

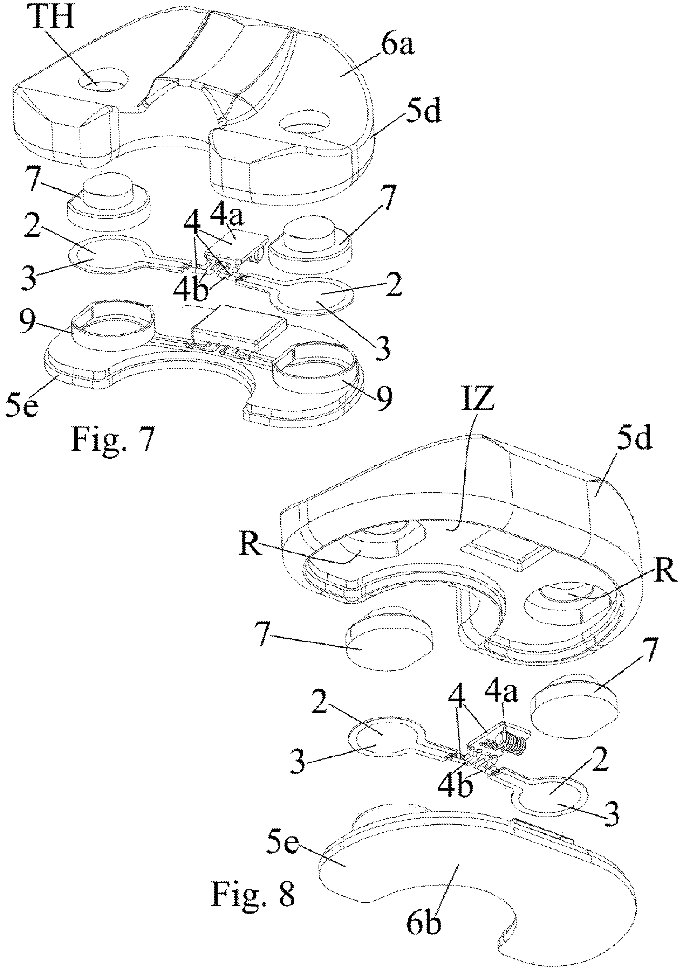

If the spacer is a knee spacer device, it comprises a tibial part 5 (see in particular figures 1 to 8) destined to be fixed to the tibia of a patient and a femoral part 50 (see in particular the figures from 9 to 13) intended to be fixed to the femur of a patient as well as to slidingly engage the tibial part 5. In this case, at least one temperature sensor 2 is in the tibial part 5 and at least one temperature sensor 2 is in the femoral part 50.

Such a spacer could be obtained, for example, as described in European patent EP1274374B1.

Thus for example the tibial part 5 has a first upper, in use, articulation surface 6a substantially curved or with a ramp delimiting a substantially concave sliding seat, while the femoral part 50 is provided with a first lower, in use, articulation face 60a substantially convex and intended to be positioned in the sliding seat for the sliding engagement of the first upper articulation surface 6a, so as to allow articulation, if desired a mutual angular or roto-translational movement between the tibial part 5 and the femoral part 50, and in turn, once the device is implanted, between the tibia and the femur.

The tibial part 5 can be substantially C-shaped.

The first articulation surface 6a can have, from one side S1 to the other S2, a raised intermediate part 5a, as well as two lowered lateral parts 5b, 5c placed one opposite to the other with respect to the intermediate part 5a. For this purpose, the tibial part 5 can be equipped with an intermediate section with a greater thickness, as well as two lateral sections with a lesser thickness placed one opposite the other with respect to the intermediate section.

In this case, a temperature sensor 2 and the electronic means 4 can be provided at any point of the tibial part 5, for example in an intermediate part of the same, if desired in the raised intermediate part 5a. In this regard, as it will be understood, infections develop in the bone interface.

As it is known, the femoral part 50 comprises two condylar sections 50a, 50b and the temperature sensor 2 comprises at least a first temperature sensor 2 in a condylar section 50a and at least a second temperature sensor 2 in the other condylar section 50b.

More specifically, the femoral part 50 can be substantially C-shaped with a base section 50c and two prongs extending from the latter and each defining a respective condylar section 50a, 50b.

The tibial part 5 and/or the femoral part 50 may or may not be made in one piece.

Moreover, the femoral part 50 can comprise a substantially curved plate-shaped body with convexity, in use, facing the tibial part 5.

The first articulation face 60a can have a central hollow band, in use destined to slidingly engage the raised intermediate part 5a of the tibial part, as well as two enlarged lateral bands placed one opposite to the other with respect to the hollow band and each one intended to engage slidingly a respective lowered lateral part 5b, 5c.

In this case, a temperature sensor 2 and the electronic means 4 can be provided at any point of the femoral part 50, for example the electronic means 4 or a respective board 4a in an intermediate part thereof with at least two temperature sensors 2 each in position proximal to a respective side of the spacer device, for example each in a respective condylar section 50a, 50b, at a free end of the base or of an intermediate portion of such condylar section 50a, 50b.

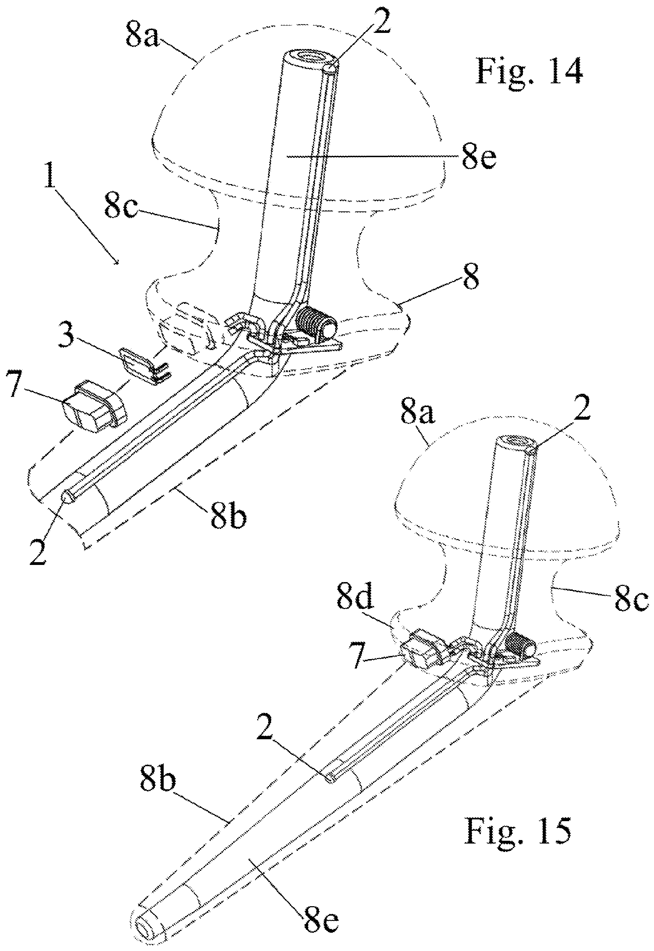

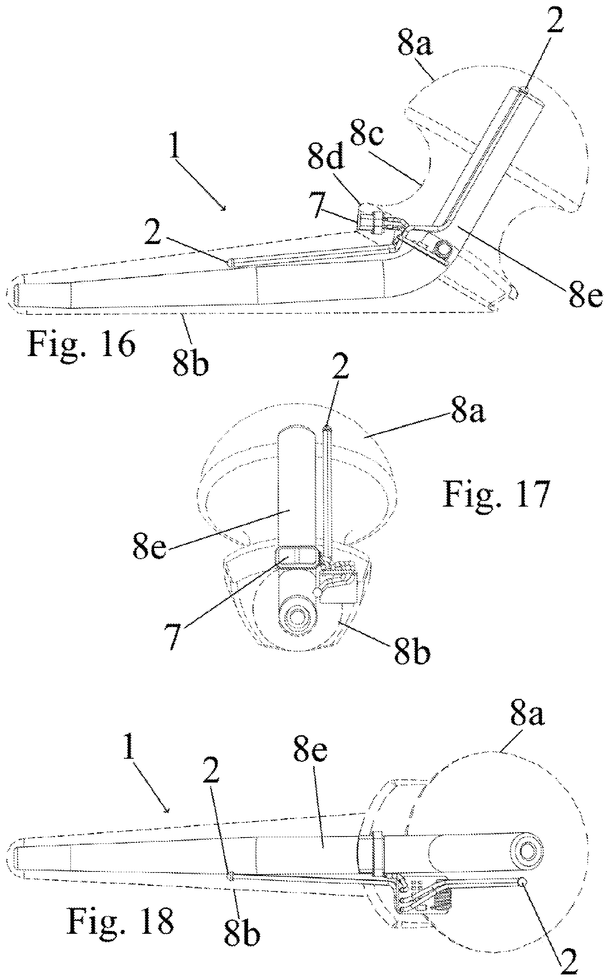

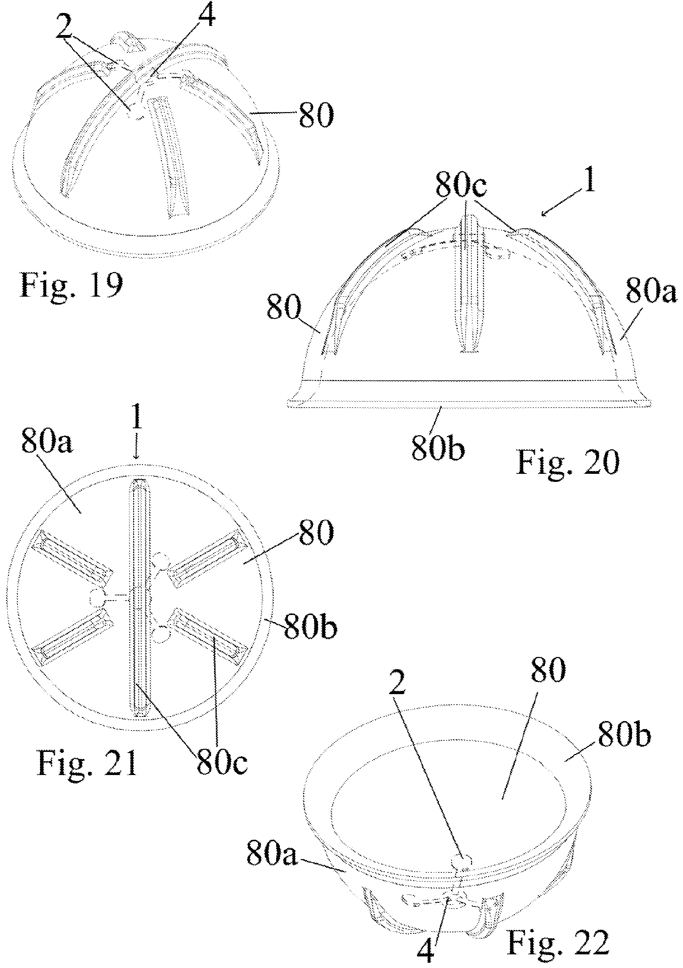

Still with reference to the presence of a temperature sensor as a means of detecting an infection, the spacer device can be a hip spacer device (see in particular figures 14 to 22), in which case the at least a temperature sensor is in an acetabular cup component 80 (figures from 19 to 22) of this device and in a femoral component 8 (figures from 14 to 18) of the device 1 designed to be inserted in the femur of a patient.

Such a femoral component 8 can be, for example, as described in the Italian patent IT1278853.

In this regard, this femoral component 8 has a head 8a, if desired hemispherically-shaped constrained, connected to or in one piece with a stem 8b, if desired with the interposition of a connecting neck section 8c. Clearly, the head 8a is arranged to be disposed, in use, within an acetabular cup, while the stem 8b would be inserted into a patient's femur.

The connecting section 8c can end in an enlarged or flanged section 8d from which the stem 8b extends.

The femoral component 8 may or may not be made in one piece.

If desired, the femoral component 8 can also have a core 8e, if desired metallic, for example rod-shaped, which extends internally from the head 8a to the stem 8b.

In this case, a temperature sensor 2 is provided at the head 8a designed to evaluate the acetabular temperature and a temperature sensor 2 at the stem 8b, designed to detect the temperature of the femoral diaphysis.

These sensors 2 as well as the transmission means 4 can be constrained, if desired by means of a specific adhesive or adhesion means, to the core 8e, if provided.

As regards instead the acetabular cup component 80, it can comprise, as is known, a cap element 80a, if desired semi-spherical, defining a concavity, which cap element 8a can have for example an open annular end 80b which is flared. Moreover, the acetabular cup component 80 can also include one or more ribs 80c, if desired lying on lines passing through the vertex of the cap element 8a, which ribs 80c clearly extend from an internal surface or in any case distal from the femoral component 8.

One or more sensors 2 (three in the example in the figures) can be provided at the vertex of the cap element 80a.

The acetabular cup component 80 may or may not be made in one piece.

With reference instead to an elbow spacer device comprising a humeral stem component and an ulnar stem component, a temperature sensor is provided in the humeral stem component and a temperature sensor is provided in the ulnar stem component.

Such a spacer could be, for example, as described in the international application published under number WO2016063155A1.

If then the spacer device was a shoulder spacer device, then a temperature sensor is in a stem component designed to be inserted into a patient's humerus and a temperature sensor is in the head component designed to be articulated with the glenoid cavity of the patient's shoulder blade.

Such a spacer can be, for example, as described in the international application published under number WO2016063146A1.

In accordance with the present invention, the sensor can comprise also or only at least one force or load sensor 3.

Such sensor can be for example a strain gauge, which if compressed changes resistance or voltage, a load cell, a piezoelectric ceramic or a transducer or a load sensor of another type.

A load sensor can be present in particular in a knee spacer device or in a hip spacer device.

The force sensor can be important for evaluating, for example, the forces applied to the spacer during walking by a patient in which the spacer is installed.

Thanks to a load sensor 3 or better to more load sensors, a spacer device according to the present invention would be able to carry out a diagnostic activity, because it can actually help the doctor to diagnose the effectiveness of the spacer for maintaining the joint space and the articulation, since if the forces applied to the spacer during walking are excessive or unbalanced, it means that the spacer is improperly implanted and thus that its effectiveness cannot be satisfactory.

With reference to the knee spacer device (see in particular figures 1 to 8) structured for example as indicated above, then the force or load sensor can be provided in the tibial part 5 and/or in the femoral part 50.

Preferably, at least two load sensors 3 are provided arranged one at one side of the spacer 1 and the other at the other side, so that the sensors 3 make it possible to evaluate any misalignments between the femoral part 50 and the tibial part 5. The two load sensors 3 are in this case advantageously arranged in a symmetric position with respect to each other with reference to a symmetry plane, in use, sagittal.

If desired, a load sensor 3 is provided in a lowered side part 5b and a load sensor 3 in the other lowered side part 5c.

More specifically, the load sensors 3 are provided at an intermediate area of the lowered side parts 5b, 5c, i.e. an area where, during the use of the spacer, contact or load applied by the femoral part is always guaranteed. In this case, the intermediate zone in which the load sensor is provided in each lowered lateral part 5b, 5c is intermediate both with reference to a front-back or anteroposterior direction, and from one side to the other or side-medial.

In this regard, considering that each lowered lateral part 5b, 5c defines a respective curved portion of the first upper articulation surface 6a with the concavity facing upwards, the positioning area of the sensors 3 is preferably that relating to the apex of the concavity or in any case to the area at the lower level of the concavity.

The load sensor 3 in the tibial part clearly guarantees a more reliable detection of the force applied on the knee, certainly more reliable than the femoral part, because in the case of the tibial part a contact and thus a load by the femoral part is always.

According to a less preferred variant, the load sensor is in the femoral part 50, for example in the base section 50c or in an intermediate portion.

If, on the other hand, the spacer device is a hip spacer device, then the load sensor 3 is preferably provided at the resting point on the diaphyseal bone, thus substantially at the point of unloading on the femur. With reference to the non-limiting embodiment shown in the figures, the load sensor is applied or provided at the connecting section 8c or better of the enlarged or flanged section 8d.

In this regard, if one put the sensor in the head of this device, contact or anyway correct detection of the load may not always be guaranteed, because when the spacer head is rotated a precise point of transferring the forces cannot would not be present. The presence of the sensor at the point of resting on the diaphyseal bone instead always guarantees the detection of the force applied on the hip.

The sensor in a device according to the present invention could also be a biochemical type sensor, for example for pH or for other parameters.

The sensor could then also include another type of sensor, if desired a sensor sensitive to biohumoral parameters such as monoclonal antibodies suitable for the supersensitive identification of bacterial presence and a bioosmotic sensor confined in the spacer device and, for example separated from the biological environment by means of a semipermeable membrane, if desired sensitive to specific ions released from any pathological process.

Moreover, the sensor could also include at least an accelerometer, designed to measure and/or detect the acceleration or movements of the spacer device and therefore of a patient in which it is implanted, for example during walking or physiotherapy sessions.

Thus, the accelerometer would collect a patient's displacement data and then allow the transmission or acquisition of the same when necessary or expected.

As it will be understood, the data collected by the accelerometer, in particular in combination with those collected by another sensor, in particular a temperature sensor, would make it possible to understand whether in the event of a parameter change, for example in the case of an increase in temperature of the spacer device, this is due to the movement of the patient and therefore of the spacer device itself or, in the absence of movement, to the onset or persistence of an infection.

A device according to the present invention comprises a main body, for example made of polymethylmethacrylate, in which case the sensor 2, 3 and the electronic means 4 are embedded in the main body, so that the sensor 2, 3 and the electronic means 4 are in a position impermeable to biological fluids of a patient.

In essence, the sensor 2, 3 as well as the electronic means 4 can be in a hermetic zone, so that these components do not come into contact with a patient's biological fluids.

A sensor 2, 3 and more particularly a load or force sensor could be encapsulated or protected in whole or in part by a sort of button component or housing 7.

This button component or housing 7, in particular in the case of a knee spacer device, moves by a very small or infinitesimal entity as a result of the force or load applied to it.

Such a button component or housing 7 could be made of any suitable material, such as for example polyethylene, ceramic or other.

According to this variant, in particular in the case of a knee spacer device, the latter delimits one or more through holes TH or in any case opening in the area of detection by means of the sensor 3, for example at the first upper articulation surface 6a, in which, in use, the push-button component or housing 7 is housed or emerges and is engageable from the outside of the spacer.

In accordance with the non-limiting embodiment shown in the figures, the spacer device 1 delimits two through holes TH one opening into a lowered side part 5b and one into another lowered part 5c, and two pressure sensors are then provided and two respective push-button components or housings 7 arranged or in any case emerging each at a respective through hole TH.

Alternatively, no button component or housing 7 could be provided, in which case the sensor 3 would be embedded in the material of the spacer device, under a layer of the same, in which case the deformation of the material, for example polymethylmethacrylate, of the device spacer would press the underlying sensor 3. In this case, the layer of the material of the spacer device above or covering the sensor could be between 0.1 and 5 mm, or between 0.1 and 3 mm.

A small gap can be delimited between the material layer of the device and the sensor.

With particular reference to a knee spacer 1, if desired to a tibial part 5 of the same, it could comprise two half-shells 5d, 5e designed to define between them a positioning zone of the sensor(s) 2, 3 and of the electronic means 4.

More specifically, a first half-shell 5d could delimit, on the one hand, the first upper articulation surface 6a and, on the other, a recessed area IZ defining inside it one or more recesses R.

The second half-shell 5e could instead define on one side a constraint or resting surface 6b on a tibia or on a component to be constrained to a tibia and on the other of the projecting parts 9 for the engagement with the recesses R.

Clearly, there could be recesses in the second half-shell 5b and protruding parts in the first half-shell 5d or an intermediate solution among those now described.

In this case, the button component(s) or housing(s) 7 and the components of the electronic means 4 and possibly the sensors 2, 3 could be housed in the recesses R of the first 5d and/or of the second 5b half-shell.

The two half-shells 5d, 5b could then be constrained to each other with any suitable means, such as bone cement, glues, adhesives or interlocking means or mechanical engagement.

Similar considerations can be made for the femoral part 50.

Clearly, only recesses could be provided in one of the half-shells in which the sensors 2, 3 and the electronic means 4 would be housed.

Advantageously, the device does not include energy sources for activating sensor 2, 3 and electronic means 4, therefore there would be a passive electronic circuit. In this case, there would be no electrical risk due to the breakdown of any battery, or short circuits or possible leakage of chemical components from the latter.

A spacer device 1 according to the present invention can be made of biologically compatible material.

This biologically compatible material can be chosen among metals, metal alloys, organo-metal compounds.

Alternatively, the biologically compatible material can be chosen from the ceramics, the high porosity resins, the plastic materials and/or a combination thereof.

Specifically, the aforementioned plastic materials can be selected from thermoplastic polymers, such as acrylic resins, polyethylene, polypropylene, polyester, thermoformable polymers and the like.

In a preferred version of the present invention, the biologically compatible material is a bone cement, for example polymethylmethacrylate (PMMA).

The presence of an internal core, for example metal, is particularly useful especially when the spacer device 1 is made of plastic or ceramic material.

In this way, it is possible to confer a better stability to the system, a high resistance to loads, etc.

In a version of the invention, the spacer device 1 is made of PMMA or the internal core is metallic and covered with PMMA.

The aforementioned biologically compatible material, thanks to its porosity, can be impregnated with pharmaceutical and/or therapeutic products, such as for example antibiotics, before its use, by the manufacturer and/or doctor before implantation.

In accordance with the present invention, a unit 10 for detecting parameters or ailments of a patient is also provided comprising a device 1 as well as a group or component for receiving and processing 11 of the data received from the sensor(s) 2, 3, which is or can be placed in electronic communication with electronic means 4.

The group for receiving and processing 11 can include an electrical power supply component of the sensor(s) 2, 3 and/or of the electronic means 4, which power supply component is separate or not integrated in the spacer device 1.

The power supply component can be integrated into a garment 12 wearable by a user, such as an elastic band or an underwear.

The group for receiving and processing 11 further comprises receiving means, such as a scanner or reader, of the data transmitted by the electronic means 4, if desired also the receiving means being integrated into the garment 12.

This group for receiving and processing 11 can then be equipped with an activation means or switch.

In essence, according to this variant, when the group for receiving and processing 11 is approached to the spacer device 1 and this group is activated, if activation is provided, the data(s) is obtained from the sensor(s), but when the group for receiving and processing 11 is moved away or switched off, then the aforementioned data(s) are no longer obtained.

The elastic band or the underwear 12 can for example comprise, as a power supply component, a built-in coil designed to induce an electric current for supplying sensor 2, 3 and electronic means 4, through the skin and tissues.

The group for receiving and processing 11 can then be provided with a control component 13 designed to receive and process the data obtained from the sensors 2, 3.

The elastic band or the underwear 12 can then comprise a data transmission system, WIFI or by cable, of the data obtained from the sensor 2, 3 to the control component 13 of the group for receiving and processing 11 for their processing.

As an alternative to what now indicated, the group for receiving and processing 11 could include a scanner or reader designed to read or receive data from the transmission means, but not a power supply component. In such a case, clearly, the spacer device 1 could include a small battery or even two or more batteries.

The control component 13 can include a computer, such as a PC, a tablet, a smartphone or other, in electronic communication with the other members of the group or component for receiving and processing 11 and responsible for processing the data received and carrying out the appropriate evaluations, for example regarding the presence or absence of the infection or the status of the infection (thanks to the temperature sensor), an excessive or unbalanced load (thanks to the load sensor) or other.

The control component 13 can be integrated in a housing of the group 11 or can be remote from it and in electronic communication, for example by cable or even wireless, with the other elements of the group.

The data collected and processed by group 11 could then be transmitted, for example, to a doctor, if desired also to the doctor's mobile phone for the appropriate evaluations.

If the device is also equipped with at least an accelerometer, then the reader would also read the data detected by the accelerometer and the control component 13, which is arranged to receive and process the data obtained from the sensors 2, 3 of the device, thanks to the data obtained from the accelerometer, could evaluate the movements of the spacer device and therefore of the patient according to the data detected by the other sensors of the device.

With regard to this, as indicated above, the data detected by the accelerometer, for example in combination with those detected by a temperature sensor, would make it possible to understand if in the event of an increase in the temperature of the spacer device, this is attributable to the patient's movement and in turn of the spacer device itself or, in the absence of movement, at the onset or persistence of an infection.

If a plurality of sensors are provided in the spacer device, in particular a plurality of load sensors, the same would be identified, i.e. they would have an individual number and the control component 13 would know the position of the sensors so as to be able to perform the appropriate assessments in a targeted or weighted manner on the actual position of any excessive temperature, or above all excessive or too low load or pressure. The group or component for receiving and processing 11 or the respective control component 13 therefore has means for identifying and recognizing the sensors 2, 3.

The computer can be equipped with specific software for data processing.

Thus, for example, the software could be designed to perform a statistical evaluation, if desired on surveys carried out several times a day, or every one, two, three or more days.

**In** this case, with reference in particular to the temperature sensor, on the basis of the finding now proposed, it would be possible, for example, to assess whether the temperature remains high or decreases over time, so as to establish in substance whether or not the infection is healing. This could be done by storing in the control component 13 or it could be associated with a data storage component where suitable values or specifications or diagrams are stored.

Therefore, the control component 13 could have a data storage space for saving the results of the processing carried out.

It is reiterated that the position of the sensor(s) is also important for assessing where an excessive load, an extremely low a load and/or an infection occurs.

As it will be ascertained, thanks to a spacer device according to the present invention it is possible to detect useful parameters regarding the implant site or the spacer itself when implanted.

Moreover, if a temperature sensor operating as indicated above is provided, it is possible to detect the permanence or healing of an infection and the possible course of the same.

A load sensor would instead allow to ascertain an incorrect positioning of the spacer or an incorrect preparation of the implant site of the same, even in the absence of symptoms detected by the patient.

In essence, thanks to the presence of one or more sensors, in particular a temperature or load sensor 3, a spacer device according to the present invention is capable of performing a diagnostic activity, since it actually can help a doctor to diagnose the progress or development of the spacer's effectiveness for reducing or eliminating an infection or for maintaining joint space.

Moreover, in particular if the spacer device does not include energy sources for the activation of the sensor(s) 2, 3 and of the electronic means 4 there would be no electrical risk due to the breaking of a possible battery, or short circuits o possible leakage of chemical components from the latter, and thus no risk of compromising the health of a patient where the spacer device is implanted would occur.

Modifications and variations of the invention are possible within the scope of protection defined by the claims.

## Claims

1. Spacer device intended to be temporarily implanted in a patient's joint area for the replacement of a joint prosthesis and for the preservation of the dimensions or spaces of the patient's joint area before the implantation of a new prosthesis, wherein said spacer device comprises at least one temperature sensor (2) as well as electronic means (4) for obtaining the data detected by said at least one temperature sensor (2),
wherein said spacer device comprises detection means of a possible infection in said spacer (1) or in the implanting area of the same, which detection means comprise said at least one temperature sensor (2),
wherein said spacer device is a knee spacer device comprising a tibial part (5) configured to be fixed to a patient's tibia and a femoral part (50) configured to be fixed to a patient's femur as well as slidingly engaging said tibial part (5) and wherein at least one temperature sensor (2) is in the tibial part (5) and at least one temperature sensor (2) is in the femoral part (50), and
wherein said femoral part (50) comprises two condylar sections (50a, 50b), **characterised in that**
said at least one temperature sensor (2) in the femoral part comprises at least one first temperature sensor in one (50a) of said condylar sections and at least one second temperature sensor (2) in the other (50b) of said condylar sections.

2. Spacer device intended to be temporarily implanted in a patient's joint area for the replacement of a joint prosthesis and for the preservation of the dimensions or spaces of the patient's joint area before the implantation of a new prosthesis, wherein said spacer device comprises at least one temperature sensor (2) as well as electronic means (4) for obtaining the data detected by said at least one temperature sensor (2),
wherein said spacer device comprises detection means of a possible infection in said spacer (1) or in the implanting area of the same, which detection means comprise said at least one temperature sensor (2), and
wherein said spacer device is a hip spacer device
and wherein at least one temperature sensor (2) is in a femoral component (8) of said spacer device configured to be inserted in the femur of a patient,
wherein said femoral component (8) comprises a head (8a) attached to a stem (8b), **characterised in that** at least one temperature sensor is in an acetabular cup component (80) of said spacer device, wherein one of the at least one temperature sensor in the femoral component (8) is provided at the head (8a), configured to evaluate acetabular temperature, and another of the at least one temperature sensor in the femoral component (8) is provided at the stem (8b), configured to detect the temperature of the femoral diaphysis.

3. Spacer device intended to be temporarily implanted in a patient's joint area for the replacement of a joint prosthesis and for the preservation of the dimensions or spaces of the patient's joint area before the implantation of a new prosthesis,
wherein said spacer device comprises at least one temperature sensor (2) as well as electronic means (4) for obtaining the data detected by said at least one temperature sensor (2),
wherein said spacer device comprises detection means of a possible infection in said spacer (1) or in the implanting area of the same, which detection means comprise said at least one temperature sensor (2), and
wherein said spacer device is an elbow spacer device, **characterised in that** the elbow spacer device comprises a humeral stem component and an ulnar stem component and wherein at least one temperature sensor is in the humeral stem component and at least one temperature sensor is in the ulnar stem component.

4. Spacer device intended to be temporarily implanted in a patient's joint area for the replacement of a joint prosthesis and for the preservation of the dimensions or spaces of the patient's joint area before the implantation of a new prosthesis,
wherein said spacer device comprises at least one temperature sensor (2) as well as electronic means (4) for obtaining the data detected by said at least one temperature sensor (2),
wherein said spacer device comprises detection means of a possible infection in said spacer (1) or in the implanting area of the same, which detection means comprise said at least one temperature sensor (2), and
wherein said spacer device is a shoulder spacer device, **characterised in that** said shoulder spacer device comprises a stem component configured to be inserted into a humerus of a patient and a head component configured to be articulated with a glenoid cavity of the patient's shoulder blade, wherein at least one temperature sensor is in the stem component and at least one temperature sensor is in the head component.

5. Spacer device according to claim 1, wherein said at least one sensor comprises at least one force or load sensor (3), and wherein said spacer device is a knee spacer device comprising a tibial part (5) intended to be fixed to a patient's tibia and a femoral part (50) intended to be fixed to a patient's femur as well as slidingly engaging said tibial part (5) and wherein said at least one force or load sensor (3) is provided in said tibial part (5) and in said femoral part (50).

6. Spacer device according to claim 5, comprising at least two load sensors (3) arranged one at one side of the spacer device and the other at the other side, so that said load sensors (3) allow to evaluate possible misalignments between femoral part (50) and tibial part (5) of the spacer device.

7. Spacer device according to claim 2, wherein said at least one sensor comprises at least one force or load sensor (3) and wherein said at least one load sensor is provided at the point of resting on the diaphyseal bone.

8. Spacer device according to claim 7, wherein the head (8a) is a constrained head (8a), connected to or in one piece with a stem (8b) with interposition of a connecting neck section (8c), wherein said load sensor (3) is applied or provided at said connecting neck section (8c) or an enlarged or flanged section (8d) of said connecting neck section (8c) from which said stem (8b) extends.

9. Spacer device according to any one of the preceding claims, comprising a main body made for example of polymethylmethacrylate and wherein said at least one sensor (2, 3) and said electronic means (4) are embedded in said main body, so that said at least one sensor (2, 3) and said electronic means (4) are in a position impermeable to biological fluids of a patient.

10. Spacer device according to any of the preceding claims, wherein said spacer device does not include energy sources for activating said at least one sensor (2, 3) and said electronic means (4).

11. Spacer device according to any one of the preceding claims, wherein said at least one sensor comprises a biochemical type sensor, a sensor sensitive to biohumoral parameters such as monoclonal antibodies suitable for the supersensitive identification of bacterial presence or a bioosmotic sensor confined in the spacer device and, for example separated from the biological environment by a semipermeable membrane, if desired sensitive to specific ions released by any pathological process.

12. Spacer device according to any of the preceding claims, wherein said at least one sensor comprises at least an accelerometer, designed to measure and/or detect the acceleration or movements of the spacer device.

13. Unit for detecting parameters or disturbances of a patient comprising a spacer device according to any of the preceding claims as well as a group or component for receiving and processing (11) the data received by said at least sensor (2, 3), which is in electronic communication with electronic means (4).

14. Unit according to claim 13, wherein said group or component for receiving and processing (11) comprises a power supply component of said at least one sensor (2, 3) and/or of said electronic means (4) separate from said device.

15. Unit according to claim 14, wherein said power supply component is integrated in a garment (12) wearable by a user.

16. Unit according to claim 13, wherein said receiving and processing group (11) includes a scanner or reader designed to read or receive data from the obtaining electronic means (4), while said spacer device (1) includes at least one battery.

17. Unit according to claim 16 when dependent on claim 12, wherein said scanner or reader is also responsible for reading the data detected by said at least one accelerometer and in which said group for receiving and processing (11) is responsible for processing the data obtained by sensors (2, 3) of said spacer device, so that thanks to the data obtained from the accelerometer it can evaluate the movements of the spacer device and therefore of the patient according to the data detected by the other sensors of the device.

18. Unit according to any one of claims 13 to 17, wherein said group for receiving and processing (11) comprises a control component (13) designed to receive and process the data obtained from said at least one sensor (2, 3) and comprises then a data transmission system, WIFI or by cable, of the data obtained from said at least one sensor (2, 3) to said control component (13) for their processing.

19. Unit according to claim 18, wherein said control component (13) includes a computer in electronic communication with the other components of the group or component for receiving and processing (11), said control component (13) being responsible for processing the data received from said at least one sensor (2, 3) and to carry out the appropriate assessments, for example on the presence or absence of the infection or the status of the infection thanks to a temperature sensor (2) or an excessive load or unbalanced load thanks to a load sensor (3)_{.}

## Patentansprüche

1. Abstandsvorrichtung, die dazu bestimmt ist, vorübergehend in den Gelenkbereich eines Patienten zur Ersetzung einer Gelenkprothese und zur Erhaltung der Abmessungen oder Abstände des Gelenkbereichs des Patienten vor der Implantation einer neuen Prothese implantiert zu werden, worin die besagte Abstandsvorrichtung mindestens einen Temperatursensor (2) sowie elektronische Mittel (4) zum Erhalten der von dem besagten mindestens einen Temperatursensor (2) erkannten Daten umfasst,
worin die besagte Abstandsvorrichtung Erfassungsmittel einer möglichen Infektion in dem besagten Abstandshalter (1) oder im Implantationsbereich desselben umfasst, wobei diese Erfassungsmittel den besagten mindestens einen Temperatursensor (2) umfassen,
worin die besagte Abstandsvorrichtung eine Knie-Abstandsvorrichtung ist, die einen Tibiateil (5) umfasst, der zur Befestigung an der Tibia eines Patienten ausgelegt ist, und einen Femurteil (50), der zur Befestigung am Femur eines Patienten ausgelegt ist sowie mit dem besagten Tibiateil (5) gleitend in Eingriff steht und worin sich mindestens ein Temperatursensor (2) im Tibiateil (5) und mindestens ein Temperatursensor (2) im Femurteil (50) befindet, und
worin der besagte Femurteil (50) zwei Kondylenabschnitte (50a, 50b) umfasst, **dadurch gekennzeichnet, dass** der besagte mindestens eine Temperatursensor (2) im Femurteil mindestens einen ersten Temperatursensor in einem (50a) der besagten Kondylenabschnitte und mindestens einen zweiten Temperatursensor (2) im anderen (50b) der besagten Kondylenabschnitte umfasst.

2. Abstandsvorrichtung, die dazu bestimmt ist, vorübergehend in den Gelenkbereich eines Patienten zur Ersetzung einer Gelenkprothese und zur Erhaltung der Abmessungen oder Abstände des Gelenkbereichs des Patienten vor der Implantation einer neuen Prothese implantiert zu werden,
worin die besagte Abstandsvorrichtung mindestens einen Temperatursensor (2) sowie elektronische Mittel (4) zum Erhalten der von dem besagten mindestens einen Temperatursensor (2) erfassten Daten umfasst,
worin die besagte Abstandsvorrichtung Erfassungsmittel einer möglichen Infektion in dem besagten Abstandshalter (1) oder im Implantationsbereich desselben umfasst, wobei diese Erfassungsmittel den besagten mindestens einen Temperatursensor (2) umfassen, und
worin es sich bei der besagten Abstandsvorrichtung um eine Hüft-Abstandsvorrichtung handelt und worin sich mindestens ein Temperatursensor (2) in einer Femurkomponente (8) der besagten Abstandsvorrichtung befindet, die zum Einsetzen im Femur eines Patienten ausgelegt ist,
worin die besagte Femurkomponente (8) einen Kopf (8a), befestigt an einem Schaft (8b), umfasst, **dadurch gekennzeichnet, dass** sich mindestens ein Temperatursensor in einer Hüftpfannenkomponente (80) der besagten Abstandsvorrichtung befindet, worin einer der mindestens einen Temperatursensoren in der Femurkomponente (8) am Kopf (8a) vorgesehen ist, der zur Bewertung der azetabulären Temperatur ausgelegt ist, und ein anderer der mindestens einen Temperatursensoren in der Femurkomponente (8) am Schaft (8b) vorgesehen ist, der zur Erfassung der Temperatur der Femurdiaphyse ausgelegt ist.

3. Abstandsvorrichtung, die dazu bestimmt ist, vorübergehend in den Gelenkbereich eines Patienten zur Ersetzung einer Gelenkprothese und zur Erhaltung der Abmessungen oder Abstände des Gelenkbereichs des Patienten vor der Implantation einer neuen Prothese implantiert zu werden,
worin die besagte Abstandsvorrichtung mindestens einen Temperatursensor (2) sowie elektronische Mittel (4) zum Erhalten der von dem besagten mindestens einen Temperatursensor (2) erfassten Daten umfasst,
worin die besagte Abstandsvorrichtung Erfassungsmittel einer möglichen Infektion in dem besagten Abstandshalter (1) oder im Implantationsbereich desselben umfasst, wobei diese Erfassungsmittel den besagten mindestens einen Temperatursensor (2) umfassen, und
worin die besagte Abstandsvorrichtung eine Ellenbogen-Abstandsvorrichtung ist, **dadurch gekennzeichnet, dass** die Ellenbogen-Abstandsvorrichtung eine humerale Schaftkomponente und eine ulnare Schaftkomponente umfasst und worin sich mindestens ein Temperatursensor in der humeralen Schaftkomponente und mindestens ein Temperatursensor in der ulnaren Schaftkomponente befindet.

4. Abstandsvorrichtung, die dazu bestimmt ist, vorübergehend in den Gelenkbereich eines Patienten zur Ersetzung einer Gelenkprothese und zur Erhaltung der Abmessungen oder Abstände des Gelenkbereichs des Patienten vor der Implantation einer neuen Prothese implantiert zu werden,
worin die besagte Abstandsvorrichtung mindestens einen Temperatursensor (2) sowie elektronische Mittel (4) zum Erhalten der von dem besagten mindestens einen Temperatursensor (2) erfassten Daten umfasst,
worin die besagte Abstandsvorrichtung Erfassungsmittel einer möglichen Infektion in dem besagten Abstandshalter (1) oder im Implantationsbereich desselben umfasst, wobei diese Erfassungsmittel den besagten mindestens einen Temperatursensor (2) umfassen, und
worin die besagte Abstandsvorrichtung eine Schulter-Abstandsvorrichtung ist, **dadurch gekennzeichnet, dass** die besagte Schulter-Abstandsvorrichtung eine Schaftkomponente, die zur Einführung in einen Humerus eines Patienten ausgelegt ist, und ein Kopfelement, das zur Gelenkverbindung mit einer Gelenkpfanne des Schulterblatts des Patienten ausgelegt ist, umfasst, worin sich mindestens ein Temperatursensor in der Schaftkomponente und mindestens ein Temperatursensor in der Kopfkomponente befindet.

5. Abstandsvorrichtung nach Anspruch 1, worin der besagte mindestens eine Sensor mindestens einen Kraft- oder Lastsensor (3) umfasst, und worin die besagte Abstandsvorrichtung eine Knie-Abstandsvorrichtung ist, umfassend einen Tibiateil (5), der zur Befestigung an der Tibia bestimmt ist, und einen Femurteil (50), der zur Befestigung am Femur eines Patienten bestimmt ist sowie mit dem besagten Tibiateil (5) gleitend in Eingriff steht, und worin der besagte mindestens eine Kraft- oder Lastsensor (3) in dem besagten Tibiateil (5) und in dem besagten Femurteil (50) vorgesehen ist.

6. Abstandsvorrichtung nach Anspruch 5, umfassend mindestens zwei Lastsensoren (3), von denen einer auf der einen Seite der Abstandsvorrichtung und der andere auf der anderen Seite angeordnet ist, sodass die besagten Lastsensoren (3) die Bewertung möglicher Fehlausrichtungen zwischen Femurteil (50) und Tibiateil (5) der Abstandsvorrichtung ermöglichen.

7. Abstandsvorrichtung nach Anspruch 2, worin der besagte mindestens eine Sensor mindestens einen Kraft- oder Lastsensor (3) umfasst und worin der besagte mindestens eine Lastsensor am Auflagepunkt auf dem diaphysealen Knochen vorgesehen ist.

8. Abstandsvorrichtung nach Anspruch 7, worin der Kopf (8a) ein festgehaltener Kopf (8a) ist, der unter Zwischensetzung eines Verbindungshalsabschnitts (8c) mit einem Schaft (8b) verbunden oder einstückig mit diesem ausgebildet ist, worin der besagte Lastsensor (3) an dem besagten Verbindungshalsabschnitt (8c) oder an einem verbreiterten oder geflanschten Abschnitt (8d) des besagten Verbindungshalsabschnitts (8c), von dem aus sich der besagte Schaft (8b) erstreckt, angebracht oder vorgesehen ist.

9. Abstandsvorrichtung nach irgendeinem der vorangegangenen Ansprüche, umfassend einen Hauptkörper, der zum Beispiel aus Polymethylmethacrylat besteht und worin der besagte mindestens eine Sensor (2, 3) und die besagten elektronischen Mittel (4) in den besagten Hauptkörper eingebettet sind, sodass der besagte mindestens eine Sensor (2, 3) und die besagten elektronischen Mittel (4) sich in einer Position befinden, die für biologische Flüssigkeiten eines Patienten undurchlässig ist.

10. Abstandsvorrichtung nach irgendeinem der vorangegangenen Ansprüche, worin die besagte Abstandsvorrichtung keine Energiequellen zur Aktivierung des besagten mindestens einen Sensors (2, 3) und der besagten elektronischen Mittel (4) umfasst.

11. Abstandsvorrichtung nach irgendeinem der vorangegangenen Ansprüche, worin der besagte mindestens eine Sensor einen Sensor biochemischer Art umfasst, einen empfindlichen Sensor für biohumorale Parameter, wie beispielsweise monoklonale Antikörper, der zur hochsensiblen Erkennung bakterieller Präsenz geeignet ist, oder einen bioosmotischen Sensor, der in der Abstandsvorrichtung untergebracht ist und, zum Beispiel getrennt von der biologischen Umgebung durch eine semipermeable Membran, falls gewünscht, für bestimmte Ionen, die bei einem beliebigen pathologischen Prozess freigesetzt werden, empfindlich ist.

12. Abstandsvorrichtung nach irgendeinem der vorangegangenen Ansprüche, worin der besagte mindestens eine Sensor mindestens einen Beschleunigungsmesser umfasst, der dazu ausgelegt ist, die Beschleunigung oder Bewegungen der Abstandsvorrichtung zu messen und/oder zu erfassen.

13. Einheit zur Erfassung von Parametern oder Störungen eines Patienten, umfassend eine Abstandsvorrichtung nach irgendeinem der vorangegangenen Ansprüche sowie eine Gruppe oder Komponente (11) zum Empfangen und Verarbeiten der von dem besagten mindestens einen Sensor (2, 3) empfangenen Daten, der in elektronischer Verbindung mit elektronischen Mitteln (4) steht.

14. Einheit nach Anspruch 13, worin die besagte Gruppe oder Komponente zum Empfangen und Verarbeiten (11) eine Stromversorgungskomponente des besagten mindestens einen Sensors (2, 3) und/oder der besagten elektronischen Mittel (4) umfasst, die von der besagten Vorrichtung getrennt ist.

15. Einheit nach Anspruch 14, worin die besagte Stromversorgungskomponente in ein von einem Benutzer tragbares Kleidungsstück (12) integriert ist.

16. Einheit nach Anspruch 13, worin die besagte Empfangs- und Verarbeitungsgruppe (11) einen Scanner oder ein Lesegerät beinhaltet, der/das dazu ausgelegt ist, Daten von den erhaltenden elektronischen Mitteln (4) zu lesen oder zu empfangen, während die besagte Abstandsvorrichtung (1) mindestens eine Batterie beinhaltet.

17. Einheit nach Anspruch 16, wenn abhängig von Anspruch 12, worin der besagte Scanner oder das besagte Lesegerät auch für das Lesen der von dem besagten mindestens einen Beschleunigungsmesser erfassten Daten zuständig ist und in der die besagte Gruppe zum Empfangen und Verarbeiten (11) für die Verarbeitung der von den Sensoren (2, 3) der besagten Abstandsvorrichtung erhaltenen Daten zuständig ist, sodass sie dank der vom Beschleunigungsmesser erhaltenen Daten die Bewegungen der Abstandsvorrichtung und somit des Patienten gemäß den von den anderen Sensoren der Vorrichtung erfassten Daten bewerten kann.

18. Einheit nach irgendeinem der Ansprüche 13 bis 17, worin die besagte Gruppe zum Empfangen und Verarbeiten (11) eine Steuerkomponente (13) umfasst, die dazu ausgelegt ist, die von dem besagten mindestens einen Sensor (2, 3) erhaltenen Daten zu empfangen und zu verarbeiten, und dann ein Datenübertragungssystem, WIFI oder Kabel, der von dem besagten mindestens einen Sensor (2, 3) erhalten Daten an die besagte Steuerkomponente (13) für deren Verarbeitung umfasst.

19. Einheit nach Anspruch 18, worin die besagte Steuerkomponente (13) einen Computer beinhaltet, der in elektronischer Verbindung mit den anderen Komponenten der Gruppe oder Komponente zum Empfangen und Verarbeiten (11) steht, wobei die besagte Steuerkomponente (13) für die Verarbeitung der von dem besagten mindestens einen Sensor empfangenen Daten (2, 3) und für die Ausführung der entsprechenden Auswertungen zuständig ist, zum Beispiel hinsichtlich des Vorhandenseins oder Nichtvorhandenseins einer Infektion oder des Infektionsstatus dank eines Temperatursensors (2) oder hinsichtlich einer Überlastung oder einer unausgeglichenen Last dank eines Lastsensors (3).

## Revendications

1. Dispositif d'espacement destiné à être implanté temporairement dans la zone articulaire d'un patient pour le remplacement d'une prothèse articulaire et pour la préservation des dimensions ou des espaces de la zone articulaire du patient avant l'implantation d'une nouvelle prothèse, dans lequel ledit dispositif d'espacement comprend au moins un capteur de température (2) ainsi que des moyens électroniques (4) pour obtenir les données détectées par ledit au moins un capteur de température (2),
dans lequel ledit dispositif d'espacement comprend des moyens de détection d'une éventuelle infection dans ledit dispositif d'espacement (1) ou dans la zone d'implantation de celui-ci, ces moyens de détection comprenant ledit au moins un capteur de température (2),
dans lequel ledit dispositif d'espacement est un dispositif d'espacement du genou comprenant une partie tibiale (5) configurée pour être fixée au tibia d'un patient et une partie fémorale (50) configurée pour être fixée au fémur d'un patient et s'engageant de manière coulissante avec ladite partie tibiale (5), et dans lequel au moins un capteur de température (2) est dans la partie tibiale (5) et au moins un capteur de température (2) est dans la partie fémorale (50), et dans lequel ladite partie fémorale (50) comprend deux sections condyliennes (50a, 50b), **caractérisées en ce que** ledit au moins un capteur de température (2) dans la partie fémorale comprend au moins un premier capteur de température dans l'une (50a) desdites sections condyliennes et au moins un deuxième capteur de température (2) dans l'autre (50b) desdites sections condyliennes.

2. Dispositif d'espacement destiné à être implanté temporairement dans la zone articulaire d'un patient pour le remplacement d'une prothèse articulaire et pour la préservation des dimensions ou des espaces de la zone articulaire du patient avant l'implantation d'une nouvelle prothèse,
dans lequel ledit dispositif d'espacement comprend au moins un capteur de température (2) ainsi que des moyens électroniques (4) pour obtenir les données détectées par ledit au moins un capteur de température (2),
dans lequel ledit dispositif d'espacement comprend des moyens de détection d'une éventuelle infection dans ledit dispositif d'espacement (1) ou dans la zone d'implantation de celui-ci, ces moyens de détection comprenant ledit au moins un capteur de température (2), et
dans lequel ledit dispositif d'espacement est un dispositif d'espacement de hanche et dans lequel au moins un capteur de température (2) est dans un composant fémoral (8) dudit dispositif d'espacement configuré pour être inséré dans le fémur d'un patient, dans lequel ledit composant fémoral (8) comprend une tête (8a) fixée à une tige (8b), **caractérisé en ce qu'**au moins un capteur de température est dans un composant de cupule acétabulaire (80) dudit dispositif d'espacement, dans lequel un dudit au moins un capteur de température dans le composant fémoral (8) est prévu au niveau de la tête (8a), configuré pour évaluer la température acétabulaire, et un autre dudit au moins un capteur de température dan le composant fémoral (8) est prévu au niveau de la tige (8b), configuré pour détecter la température de la diaphyse fémorale.

3. Dispositif d'espacement destiné à être implanté temporairement dans la zone articulaire d'un patient pour le remplacement d'une prothèse articulaire et pour la préservation des dimensions ou des espaces de la zone articulaire du patient avant l'implantation d'une nouvelle prothèse,
dans lequel ledit dispositif d'espacement comprend au moins un capteur de température (2) ainsi que des moyens électroniques (4) pour obtenir les données détectées par ledit au moins un capteur de température (2),
dans lequel ledit dispositif d'espacement comprend des moyens de détection d'une éventuelle infection dans ledit dispositif d'espacement (1) ou dans la zone d'implantation de celui-ci, ces moyens de détection comprenant ledit au moins un capteur de température (2), et
dans lequel ledit dispositif d'espacement est un dispositif d'espacement du coude, **caractérisé en ce que** le dispositif d'espacement du coude comprend un composant de tige humérale et un composant de tige ulnaire et dans lequel au moins un capteur de température est situé dans le composant de tige humérale et au moins un capteur de température est dans le composant de tige ulnaire.

4. Dispositif d'espacement destiné à être implanté temporairement dans la zone articulaire d'un patient pour le remplacement d'une prothèse articulaire et pour la préservation des dimensions ou des espaces de la zone articulaire du patient avant l'implantation d'une nouvelle prothèse,
dans lequel ledit dispositif d'espacement comprend au moins un capteur de température (2) ainsi que des moyens électroniques (4) pour obtenir les données détectées par ledit au moins un capteur de température (2),
dans lequel ledit dispositif d'espacement comprend des moyens de détection d'une éventuelle infection dans ledit dispositif d'espacement (1) ou dans la zone d'implantation de celui-ci, ces moyens de détection comprenant ledit au moins un capteur de température (2), et
dans lequel ledit dispositif d'espacement est un dispositif d'espacement d'épaule, **caractérisé en ce que** ledit dispositif d'espacement d'épaule comprend un composant de tige configuré pour être inséré dans l'humérus d'un patient et un composant de tête configuré pour être articulé avec una cavité glénoïde de l'omoplate du patient, dans lequel au moins un capteur de température est dans le composant de tige et au moins un capteur de température est dans le composant de tête.

5. Dispositif d'espacement selon la revendication 1, dans lequel ledit au moins un capteur comprend au moins un capteur de force ou de charge (3), et dans lequel ledit dispositif d'espacement est un dispositif d'espacement de genou comprenant une partie tibiale (5) destinée à être fixée au tibia d'un patient et une partie fémorale (50) destinée à être fixée au fémur d'un patient et à s'engager aussi de manière coulissante dans ladite partie tibiale (5), et dans lequel ledit au moins un capteur de force ou de charge (3) est prévu dans ladite partie tibiale (5) et dans ladite partie fémorale (50).

6. Dispositif d'espacement selon la revendication 5, comprenant au moins deux capteurs de charge (3) disposés l'un d'un côté du dispositif d'espacement et l'autre de l'autre côté, de sorte que lesdits capteurs de charge (3) permettent d'évaluer des désalignements éventuels entre partie fémorale (50) et partie tibiale (5) du dispositif d'espacement.

7. Dispositif d'espacement selon la revendication 2, dans lequel ledit au moins un capteur comprend au moins un capteur de force ou de charge (3) et dans lequel ledit au moins un capteur de charge est situé au point de repos sur l'os diaphysaire.

8. Dispositif d'espacement selon la revendication 7, dans lequel la tête (8a) est une tête contrainte (8a), reliée à ou en une seule pièce avec une tige (8b) avec interposition d'une section de col de connexion (8c), dans lequel ledit capteur de charge (3) est appliqué ou prévu sur ladite section de col de connexion (8c) ou sur une section élargie ou à bride (8d) de ladite section de col de connexion (8c) à partir de laquelle ladite tige (8b) s'étend.

9. Dispositif d'espacement selon l'une quelconque des revendications précédentes, comprenant un corps principal réalisé par exemple en polyméthacrylate de méthyle et dans lequel au moins un capteur (2, 3) et lesdits moyens électroniques (4) sont intégrés audit corps principal, de sorte que ledit au moins un capteur (2, 3) et lesdits moyens électroniques (4) soient dans une position imperméable aux fluides biologiques d'un patient.

10. Dispositif d'espacement selon l'une quelconque des revendications précédentes, dans lequel ledit dispositif d'espacement ne comprend pas de sources d'énergie pour activer ledit au moins un capteur (2, 3) et lesdits moyens électroniques (4).

11. Dispositif d'espacement selon l'une quelconque des revendications précédentes, dans lequel ledit au moins un capteur comprend un capteur de type biochimique, un capteur sensible aux paramètres biohumoraux tels que les anticorps monoclonaux adaptés à l'identification ultrasensible de présence bactérienne ou un capteur bioosmotique confiné dans le dispositif d'espacement et, par exemple, séparé de l'environnement biologique par une membrane semi-perméable, si souhaité sensible aux ions spécifiques libérés par tout processus pathologique.

12. Dispositif d'espacement selon l'une quelconque des revendications précédentes, dans lequel ledit au moins un capteur comprend au moins un accéléromètre, conçu pour mesurer et/ou détecter l'accélération ou les mouvements du dispositif d'espacement.

13. Unité de détection des paramètres ou des perturbations d'un patient comprenant un dispositif d'espacement selon l'une quelconque des revendications précédentes ainsi qu'un groupe ou composant pour recevoir et traiter (11) les données reçues par ledit au moins un capteur (2, 3), qui est en communication électronique avec des moyens électroniques (4).

14. Unité selon la revendication 13, dans laquelle ledit groupe ou composant de réception et de traitement (11) comprend un composant d'alimentation dudit au moins un capteur (2, 3) et/ou desdits moyens électroniques (4) distinct dudit dispositif.

15. Unité selon la revendication 14, dans laquelle ledit composant d'alimentation est intégré dans un vêtement (12) pouvant être porté par un utilisateur.

16. Unité selon la revendication 13, dans laquelle ledit groupe de réception et de traitement (11) comprend un scanner ou un lecteur conçu pour lire ou recevoir des données provenant des moyens électroniques d'obtention (4), tandis que ledit dispositif d'espacement (1) comprend au moins une batterie.

17. Unité selon la revendication 16 lorsqu'elle dépend de la revendication 12, dans laquelle ledit scanner ou lecteur est également responsable de la lecture des données détectées par ledit au moins un accéléromètre et dans laquelle ledit groupe de réception et de traitement (11) est responsable du traitement des données obtenues par les capteurs (2, 3) dudit dispositif d'espacement, de sorte que grâce aux données obtenues de l'accéléromètre, il peut évaluer les mouvements du dispositif d'espacement et donc du patient selon les données détectées par les autres capteurs du dispositif.

18. Unité selon l'une quelconque des revendications 13 à 17, dans laquelle ledit groupe de réception et de traitement (11) comprend un composant de commande (13) conçu pour recevoir et traiter les données obtenues à partir dudit au moins un capteur (2, 3) et comprend ensuite un système de transmission de données, WIFI ou par câble, des données obtenues à partir dudit au moins un capteur (2, 3) audit composant de commande (13) pour leur traitement.

19. Unité selon la revendication 18, dans laquelle ledit composant de commande (13) comprend un ordinateur en communication électronique avec les autres composants du groupe ou composant de réception et de traitement (11), ledit composant de commande (13) étant responsable du traitement des données reçues dudit au moins un capteur (2, 3) et de la réalisation des évaluations appropriées, par exemple sur la présence ou l'absence de l'infection ou l'état de l'infection grâce à un capteur de température (2) ou une charge excessive ou une charge déséquilibrée grâce à un capteur de charge (3).
